# EUROPÄISCHE PATENTSCHRIFT

(11) **EP 0 562 345 B1**
(45) Veröffentlichungstag und Bekanntmachung des Hinweises auf die Patenterteilung: **20.05.1998**
(21) Anmeldenummer: 93103717.0
(22) Anmeldetag: 09.03.1993
(51) Int. Cl.: C07D 233/58

(54) **Verfahren zur Herstellung von 4-substituierten Imidazolen**
Process for the preparation of 4-substituted imidazoles
Procédé de préparation d'imidazoles substitués en 4

(30) Priorität: 26.03.1992 DE 4209847
(43) Veröffentlichungstag der Anmeldung: 29.09.1993
(73) Patentinhaber: BASF Aktiengesellschaft, 67063 Ludwigshafen (DE)
(72) Erfinder: Koehler, Ulrich, Dr., W-6800 Mannheim 51 (DE); Kahl, Thomas-Michael, Dr., W-6725 Roemerberg (DE); Neuhauser, Horst, Dr., W-6724 Dudenhofen (DE); Siegel, Hardo, Dr., W-6720 Speyer (DE); Kroener, Michael, Dr., W-6800 Mannheim 31 (DE)

(56) Entgegenhaltungen:
- EP-A- 0 000 208
- EP-A- 0 025 191
- EP-A- 0 036 519
- EP-A- 0 039 828
- EP-A- 0 185 962
- US-A- 3 629 278
- CHEMICAL ABSTRACTS, vol. 103, no. 17, 28. Oktober 1985, Columbus, Ohio, US; abstract no. 141965a, '4-Methylimidazole' & JP-A-60 105 664 (TAOKA CHEMICAL CO.)

## Beschreibung

Die vorliegende Erfindung betrifft ein Verfahren zur Herstellung von 4-substituierten Imidazolen durch katalytische Hydrierung von N-Formyl-α-aminonitrilen bei erhöhten Drücken an Hydrierkatalysatoren und anschließende Umsetzung der erhaltenen N-Formyl-1,2-diamine an Cyclisierungs-/Dehydrierungskatalysatoren bei erhöhten Temperaturen.

Aus der DE-A-30 09 605 und der EP-A-25 191 ist ein Verfahren zur Herstellung von Imidazolen durch intramolekularen Ringschlußreaktion mit anschließender Dehydrierung von N,N'-Bis-formyl-1,2-diaminen mit Ni/Mo, Co/Mo bzw. Zn-Katalysatoren in der Gasphase bekannt. Hierin wird 4-Methylimidazol mit ca. 70 % Ausbeute erhalten. Ein Nachteil dieses Verfahrens ist jedoch, daß man in einer vorgeschalteten Stufe zunächst die Bisformylverbindung herstellen muß (z.B. mit Ameisensäuremethylester) und man bei der anschließenden Cyclisierung wieder eine Formylgruppe (als CO) verliert.

Der zuvor genannte Nachteil einer mehrstufigen Synthese besteht umsomehr, wenn die Verfahren auf einer vorgeschalteten Herstellung von Imidazolinen beruhen (DE-A-27 48 976; DE-A-30 09 631; US-A-4 927 942).

Der vorliegenden Erfindung lag daher die Aufgabe zugrunde, den zuvor genannten Nachteilen abzuhelfen.

Demgemäß wurde ein neues und verbessertes Verfahren zur Herstellung von 4-substituierten Imidazolen der allgemeinen Formel I 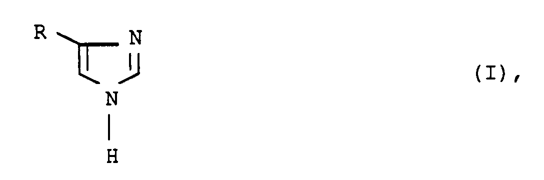 in der
- R: C₁- bis C₂₀-Alkyl, C₃- bis C₂₀-Cycloalkyl, Aryl, C₇- bis C₂₀-Aralkyl,
bedeutet, gefunden, welches dadurch gekennzeichnet ist, daß man
a) N-Formyl-α-aminonitrile der allgemeinen Formel II 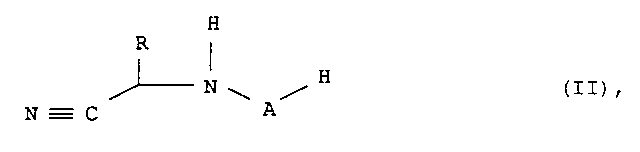 in der R die oben genannten Bedeutungen hat und A für Carbonyl steht, bei Temperaturen von 20 bis 200°C und Drücken von 20 bis 500 bar mit Wasserstoff in Gegenwart eines Hydrierkatalysators und
b) die erhaltenen N-Formyl-1,2-diamine der allgemeinen Formel III 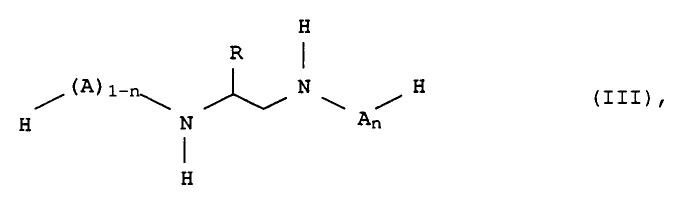 in der R und A die oben genannten Bedeutungen haben und n für 0 oder 1 steht, an Cyclisierungs-/Dehydrierungskatalysatoren bei Temperaturen von 200 bis 600°C und Drücken von 0,001 bis 5 bar umsetzt.

Das erfindungsgemäße Verfahren kann wie folgt durchgeführt werden:
a) Die für das Verfahren eingesetzten N-Formyl-α-aminonitrile II sind leicht zugängliche Verbindungen und können auf einfachem Wege z.B. nach DE-A-19 50 280 durch Cyanhydrinsynthese hergestellt werden.
   Die Hydrierung der Nitrilgruppe kann sowohl mit komplexen Metallhydriden wie Lithiumtrialkoxyaluminiumhydrid (vgl. Milôs Hudlick'y, Red. in Org. Chemistry, Ellis Horwood Ltd., 1986, S. 173 bis 175), als auch bevorzugt katalytisch mit Heterogenkatalysatoren z.B. Metallen wie Fe, Co, Ni, Pt, Pd, Rh, Ru vorgenommen werden. Im Falle der heterogen-katalysierten Hydrierung arbeitet man bevorzugt bei höheren Wasserstoffdrücken in der Flüssigphase bei Drücken von 20 bis 500 bar, besonders bevorzugt 50 bis 300 bar und Temperaturen von 20 bis 200°C, besonders bevorzugt 40 bis 120°C). Zu hohe Temperaturen und Reaktionsdauern sind zu vermeiden, um eine Entformylierung zu verhindern.
   Die erfindungsgemäß verwendeten Katalysatoren können sowohl als Trägerkatalysatoren oder in kompakter Form, d.h. ohne Träger eingesetzt werden. Die Art des Trägermaterials ist in der Regel nicht kritisch. Es können übliche Trägermaterialien wie Siliciumdioxid, Aluminiumoxide, Titandioxide, Aktivkohle, Silikate oder Zeolithe verwendet werden. Erforderlichenfalls können zur Herstellung der Katalysatoren Bindemittel oder Formhilfsmittel mitverwendet werden.
   Die Katalysatoren werden vorzugsweise vor ihrem Einsatz in aller Regel mit Wasserstoff aktiviert. Dabei werden die nach der Calcinierung normalerweise in Form ihrer Oxide vorliegenden aktiven Katalysatorbestandteile größtenteils reduziert und zwar in der Regel zu den entsprechenden Metallen. Weitere Einzelheiten zur Herstellung dieser Katalysatoren können der DE-A 23 21 101 sowie der DE-A 39 04 083 entnommen werden.
   Die katalytische Hydrierung der N-Formyl-a-aminonitrile II mittels Wasserstoff und Heterogenkatalysatoren kann auf diskontinuierliche Weise in Rührkesseln oder auf kontinuierliche Weise in Rührkesselkaskaden oder in Rohrreaktoren an sich herkömmlicher Bauart, die in der Sumpf- oder Rieselfahrweise betrieben werden können, erfolgen. Dabei kann der Hydrierkatalysator in der Reaktionsmischung suspendiert, oder aber in einer Festbettanordnung im Reaktor angebracht werden. Die Hydrierung kann in einem Lösungsmittel, z.B. Toluol, Xylol, Tetrahydrofuran, Dioxan, Methanol, Ethanol, Diethylether, Methyl-tert.-butylether, Dimethylformamid, N-Methylpyrrolidon und anderen in Abwesenheit oder bevorzugt in Anwesenheit von 1 bis 50 Mol-% Ammoniak pro Mol II stattfinden. Die Anwesenheit eines Lösungsmittels ist für die Durchführung der Hydrierung nicht erforderlich. Besonders bevorzugt als katalytisch wirksame Metalle sind Fe, Co und Ni.
b) Vor der Umsetzung zu den 4-substituierten Imidazolen I ist eine destillative Abtrennung des Lösungsmittels sowie gegebenenfalls eine z.B. destillative Reinigung des Hydrieraustrages möglich, aber nicht unbedingt erforderlich. Bestimmte Lösungsmittel, die in der Gasphase zu Alkylierungen neigen (z.B. Methanol, Ethanol) sollten jedoch entfernt werden.
   Um ein Auskristallisieren der gebildeten Imidazole I nach Austritt aus der Reaktionszone zu vermeiden, kann es zweckmäßig sein, die eingesetzten N-Formyl-1,2-diamine III mit einem unter den Reaktionsbedingungen weitgehend inerten Lösungsmittel zu verdünnen.
   Geeignete unter den Reaktionsbedingungen weitghend inerte Lösungsmittel sind z.B. Wasser oder Ether wie Tetrahydrofuran und Dioxan.
   Die Cyclisierungs-/Dehydrierungsreaktion kann bevorzugt in einem Gasphasen-Festbett- oder Wirbelbettreaktor durchgeführt werden. Prinzipiell ist die Reaktion aber auch in der Flüssigphase möglich. Für die bevorzugte Gasphasenfahrweise arbeitet man bei Temperaturen zwischen 200 und 600°C, bevorzugt 280 und 450°C und Drücken von 0,001 bis 5 bar, bevorzugt 0,02 bis 1,5 bar, besonders bevorzugt bei Atmosphärendruck (Normaldruck), gegebenenfalls im H₂- oder H₂/CO-Gasstrom. Es ist natürlich möglich, vor die eigentliche Dehydrierungsreaktion einen Cyclisierungsschritt an einem sauren Katalysator zum Imidazolin zu schalten, z.B. unter Einsatz von sauren Heterogenkatalysatoren wie Zeolithen, z.B. Aluminiumsilikatzeolithe, Borsilikatzeolithe, Eisensilikatzeolithe, Galliumsilikatzeolithe und Silikalithe des Pentasil-, Faujasit-, X-, Y-Typs oder sauren Metallsalzen und sauren Metalloxiden z.B. Phosphate, Hydrogenphosphate, Dihydrogenphosphate, Pyrrophosphate und beliebige andere Phosphate der Elemente Ca, Ba, Sr, Al, Fe und Zr mit und ohne Zusätze von SiO₂, TiO₂, Al₂O₃ (auch als Trägermaterial) und Oxide der Elemente Mo und W. Geeignet sind hierzu z.B. auch die in EP-A-12 371 aufgeführten Katalysatoren.
   Sehr viel ökonomischer und im Sinne der vorliegenden Erfindung ist es jedoch, die Cyclisierung und Dehydrierung der N-Formyl-1,2-diamine III in einem Schritt durchzuführen. Geeignete Katalysatoren sind die im Prinzip literaturbekannten Dehydrierungskatalysatoren, die Elemente der Nebengruppen IVb, Vb, VIb, VIIb, VIII, Ib und IIb enthalten, wie z. B. Cr, Mo, Fe, Ru, Co, Rh, Ni, Pd, Pt, Cu, Ag, Zn, Cd. Bevorzugt sind Ni, Co, Fe, Mo, Zn, Cu, Ag, Cr, Pt, Pd oder deren Mischungen. Es können Voll- oder Trägerkatalysatoren eingesetzt werden. Setzt man Trägerkatalysatoren ein, so kommen als geeignete Trägermaterialien neben dem bereits bei den Hydrierkatalysatoren (s.o.) genannten auch Erdalkalioxide und Erdalkalicarbonate, Lantanoxide und Lantancarbonate in Frage. Unter Umständen kann der Einsatz von sauren Trägermaterialien (wie oben beschrieben) Raum-Zeit-Ausbeute und Selektivität begünstigen. Die katalytisch aktiven Komponenten können als Oxide (z.B. ZnO) oder in ihrer metallischen Form (z.B. Pd) verwendet werden. Bei Verwendung der metallischen Form ist eine Aktivierung des Metalls im Wasserstoffstrom für einige Stunden bei Temperaturen zwischen 250 und 600°C sinnvoll.
   Das durch Cyclisierung und Dehydrierung gewonnene Rohprodukt kann schließlich destillativ von einem evtl. zugesetzten Lösungsmittel befreit und durch Destillation, Kristallisation oder Chromatographie gereinigt werden.

Die Substituenten R und A sowie der Index n in den Verbindungen I, II und III haben die folgenden Bedeutungen:
- R: - C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- A: - Carbonyl
- n: - 0 oder 1.

4-substituierte Imidazole I sind wichtige Zwischenprodukt z.B. für die Herstellung von Pharmawirkstoffen (z.B. Cimetidin).

### Beispiele

### Beispiel 1

### Hydrierung von N-Formylalaninnitril

In einem 10-l-Rührautoklav wurden 200 g Raney-Nickel und 1000ml Tetrahydrofuran vorgelegt und nach Spülen mit Stickstoff 1275 g Ammoniak (75 mol) zugegeben. Man preßte 30 bar Wasserstoff auf, erwärmte auf 60°C und erhöht den Druck auf 200 bar H₂. Bei diesen Bedingungen wurden innerhalb von 10 h 2000 g (20,4 mol) N-Formylalaninnitril zugepumpt und noch 12 h bei 250 bar H₂ gerührt.

Nach Abkühlen und Entspannen wurde der Austrag über eine Druckfilternutsche filtriert und i. Vak. eingeengt. Man erhielt 1980 g (95 %) Rohaustrag mit einer Reinheit von 96 % (GC).

Ein Produkt mit einer Reinheit > 99 % erhielt man durch kontinuierliche Destillation im Dünnfilmverdampfer (Sdp. 115°C/0,3 mbar). Eine solche Reinigung ist jedoch für die nachfolgende Cyclisierung / Dehydrierung nicht notwendig.

### Beispiel 2

### Herstellung von 4-Methylimidazol aus N-Formyl-1,2-propandiamin

In einen elektrisch beheizbaren 250-ml-Festbettreaktor wurden 200ml eines Katalysators gefüllt, der aus 0,5 Pd auf 80 % Al₂O₃ und 20 % CaO bestand. Der Katalysator wurde 12 h bei 350 bis 375°C im Wasserstoffstrom (ca. 30 l/h) aktiviert.

Anschließend wurden 100 g/h eines Gemisches, das zu 50 % aus Wasser und zu 50 % aus N-Formyl-1,2-propandiamin bestand, dampfförmig bei Normaldruck zusammen mit ca. 50 l/h Wasserstoff bei 350 bis 375°C über den Katalysator geleitet. Der Austrag wurde kondensiert und gesammelt und betrug nach 3h ca. 280 g Rohprodukt, das in einer 20cm-Füllkörperkolonne fraktioniert destilliert wurde. Man erhielt 115,7g (96%) 4-Methylimidazol; Sdp.: 136 bis 140°C/12 mbar; > 99 % (GC), das beim Abkühlen zu einem farblosen Feststoff erstarrte.

Die Gesamtausbeute aus Beispiel 1 und 2 lag bei 91,2 %.

### Beispiel 3

Es wurde wie in Beispiel 2 verfahren, die Katalysatorfüllung bestand jedoch aus einem Katalysator, der zu 50 % aus CuO und 50 % aus Al₂O₃ zusammengesetzt war. Aus 50 g/h N-Formylalaninnitril (kein Lösungsmittel) wurden nach 3 h 135 g Rohprodukt erhalten. Durch fraktionierte Destillation wurden daraus 117,9 g (97,8 %) 4-Methylimidazol der Reinheit > 99 % (GC) gewonnen. Dies entspricht einer Gesamtausbeute (einschl. Beispiel 1) von 92,9 %.

### Beispiel 4

Es wurde wie in Beispiel 3 verfahren, die Katalysatorfüllung bestand jedoch aus einem Katalysator, der zu 80 % aus ZnO und 20 % aus Al₂O₃ zusammengesetzt war. Der Katalysator wurde vor Gebrauch nicht reduziert. Nach 3 h Betriebsdauer unter den Bedingungen von Beispiel 3 erhielt man nach Destillation 102,3 g (84,9 %) 4-Methylimidazol der Reinheit > 99 % (GC). Dies entspricht einer Gesamtausbeute (einschl. Beispiel 1) von 80,7 %.

## Patentansprüche

1. Verfahren zur Herstellung von 4-substituierten Imidazolen der allgemeinen Formel I 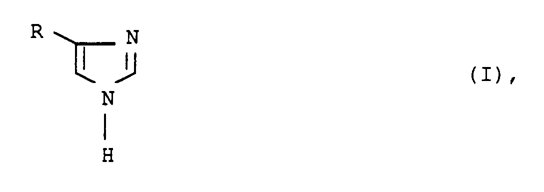 in der
R C₁- bis C₂₀-Alkyl, C₃- bis C₂₀-Cycloalkyl, Aryl, C₇- bis C₂₀-Aralkyl,
bedeutet, dadurch gekennzeichnet, daß man
a) N-Formyl-α-aminonitrile der allgemeinen Formel II 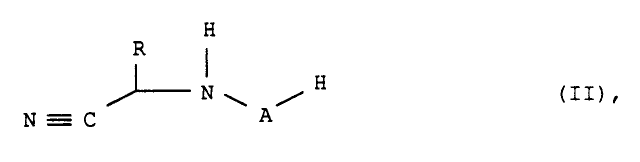 in der R die oben genannten Bedeutungen hat und A für Carbonyl steht, bei Temperaturen von 20 bis 200°C und Drücken von 20 bis 500 bar mit Wasserstoff in Gegenwart eines Hydrierkatalysators und
b) die erhaltenen N-Formyl-1,2-diamine der allgemeinen Formel III 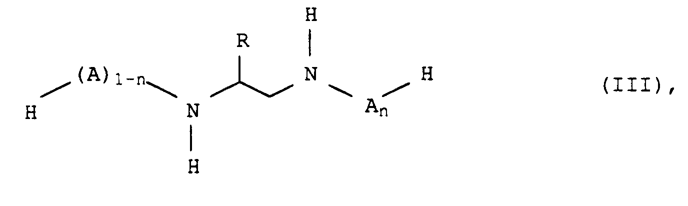 in der R und A die oben genannten Bedeutungen haben und n für 0 oder 1 steht, an Cyclisierungs-/Dehydrierungskatalysatoren bei Temperaturen von 200 bis 600°C und Drücken von 0,001 bis 5 bar umsetzt.

2. Verfahren zur Herstellung von 4-substituierten Imidazolen I nach Anspruch 1, dadurch gekennzeichnet, daß man Hydrierkatalysatoren mit Fe, Co, Ni, Pd, Pt, Rh und/oder Ru als katalytisch aktive Masse verwendet.

3. Verfahren zur Herstellung von 4-substituierten Imidazolen I nach Anspruch 1, dadurch gekennzeichnet, daß man Cyclisierungs-/Dehydrierungskatalyastoren mit einem oder mehreren Elementen der Nebengruppen IVb, Vb, VIb, VIIb, VIII, Ib und IIb des Periodensystems der Elemente verwendet.

4. Verfahren zur Herstellung von 4-substituierten Imidazolen I nach Anspruch 1, dadurch gekennzeichnet, daß man das N-Formyl-α-aminonitril II, in dem R für Methyl steht, mit einem Fe-, Co- und/oder Ni-Katalysator in der Flüssigphase hydriert und an einem Katalysator, der eines oder mehrere Elemente von Fe, Co, Ni, Pd, Pt, Cu, Ag, Zn, Cr, Mo enthält, in der Gasphase cyclisiert und dehydriert.

## Claims

1. A process for preparing 4-substituted imidazoles of the formula I 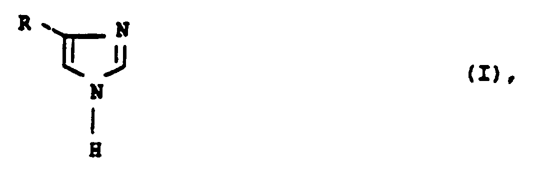 where
R is C₁-C₂₀-alkyl, C₃-C₂₀-cycloalkyl, aryl, C₇-C₂₀-aralkyl,
which comprises reacting
a) N-formyl-α-amino nitriles of the formula II 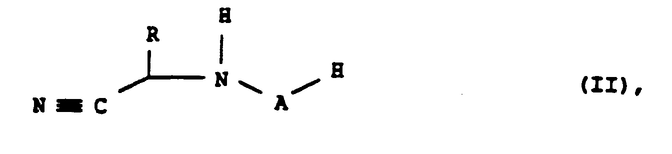 where R has the abovementioned meanings, and A is carbonyl, at from 20 to 200°C under pressures of from 20 to 500 bar with hydrogen in the presence of a hydrogenation catalyst and
b) the resulting N-formyl-1,2-diamines of the formula III 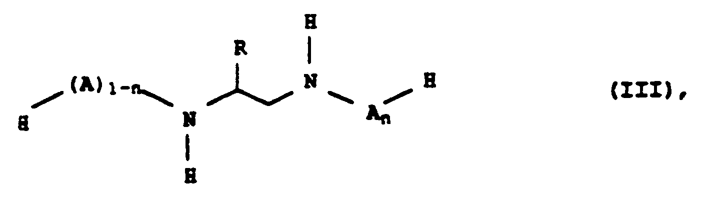 where R and the A have the abovementioned meanings, and n is 0 or 1, on cyclization/dehydrogenation catalysts at from 200 to 600°C under pressures of from 0.001 to 5 bar.

2. A process for preparing 4-substituted imidazoles I as claimed in claim 1, wherein hydrogenation catalysts having Fe, Co, Ni, Pd, Pt, Rh and/or Ru as catalytically active mass are used.

3. A process for preparing 4-substituted imidazoles I as claimed in claim 1, wherein cyclization/dehydrogenation catalysts having one or more elements of subgroups IVb, Vb, VIb, VIIb, VIII, Ib and IIb of the Periodic Table of the Elements are used.

4. A process for preparing 4-substituted imidazoles I as claimed in claim 1, wherein the N-formyl-α-amino nitrile II where R is methyl is hydrogenated using an Fe, Co and/or Ni catalyst in the liquid phase, and cyclized and dehydrogenated on a catalyst which contains one or more elements from Fe, Co, Ni, Pd, Pt, Cu, Ag, Zn, Cr, Mo in the gas phase.

## Revendications

1. Procédé de préparation d'imidazoles substitués en position 4 de formule générale I 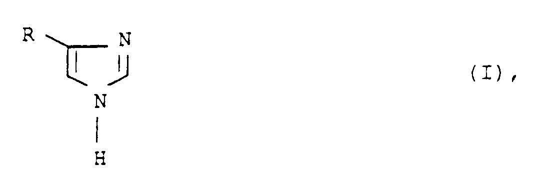 dans laquelle
R représente un groupement alkyle en C₁-C₂₀, cycloalkyle en C₃-C₂₀, aryle, aralkyle en C₇-C₂₀,
caractérisé en ce que l'on fait réagir
a) des N-formyl-α-aminonitriles de formule générale II 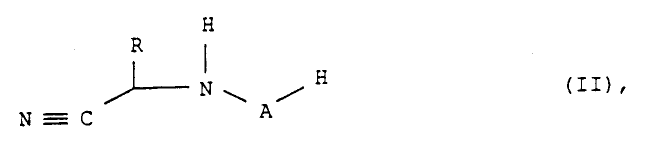 dans laquelle R prend les significations susmentionnées et A est mis pour un groupement carbonyle, à des températures de 20-200°C et sous des pressions de 20-500 bar avec de l'hydrogène en présence d'un catalyseur d'hydrogénation et
b) les N-formyl-1,2-diamines obtenues de formule générale III 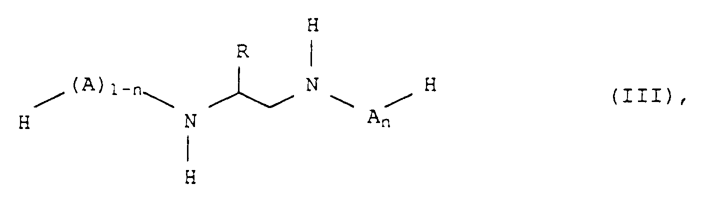 dans laquelle R et A ont les significations susmentionnées, et n est mis pour 0 ou 1, sont mises à réagir en présence de catalyseurs de cyclisation/déshydrogénation à des températures de 200-600°C et sous des pressions de 0,001 à 5 bar.

2. Procédé de préparation d'imidazoles I substitués en position 4 selon la revendication 1, caractérisé en ce que l'on utilise des catalyseurs d'hydrogénation contenant comme masse à action catalytique Fe, Co, Ni, Pd, Pt et/ou Ru.

3. Procédé de préparation d'imidazoles I substitués en position 4 selon la revendication 1, caractérisé en ce que l'on utilise des catalyseurs de cyclisation/déshydrogénation contenant un ou plusieurs éléments des groupes IVb, Vb, VIb, VIIb, VIII, Ib, IIb, de la classification périodique des éléments.

4. Procédé de préparation d'imidazoles I substitués en position 4 selon la revendication 1, caractérisé en ce que l'on hydrogène en phase liquide le N-formyl-α-aminonitrile II dans lequel R est mis pour un groupement méthyle, avec un catalyseur au Fe, Co et/ou Ni, et en ce qu'on le cyclise et le déshydrogène en phase gazeuse avec un catalyseur contenant un ou plusieurs éléments parmi Fe, Co, Ni, Pd, Pt, Cu, Ag, Zn, Cr, Mo.
